(19) Europäisches Patentamt
European Patent Office
Office européen des brevets

(11) Veröffentlichungsnummer: **0 007 101**
**B1**

# (12) EUROPÄISCHE PATENTSCHRIFT

(45) Veröffentlichungstag der Patentschrift: **26.08.81**

(51) Int. Cl.³: **C 07 C 31/18**, C 07 C 29/136, C 08 G 18/32

(21) Anmeldenummer: **79102407.8**

(22) Anmeldetag: **12.07.79**

(54) **Verfahren zur Herstellung von Polyalkoholen sowie Verfahren zur Herstellung von Polyurethan-Kunststoffen unter Verwendung dieser Polyalkohole.**

(30) Priorität: **19.07.78 DE 2831659**

(43) Veröffentlichungstag der Anmeldung: **23.01.80 Patentblatt 80/2**

(45) Bekanntmachung des Hinweises auf die Patenterteilung: **26.08.81 Patentblatt 81/34**

(84) Benannte Vertragsstaaten: **BE DE FR GB IT**

(56) Entgegenhaltungen:
**DE-A1-2 536 416**
**DE-A1-2 639 083**
**DE-A1-2 639 084**
**DE-B-1 069 135**
**DE-B-1 543 178**
**DE-C-830 951**
**DE-C-888 096**
**FR-A-835 752**
**GB-A-1 497 318**
**US-A-2 224 910**
**US-A-2 271 083**
**US-A-2 775 621**
**US-A-3 055 840**
**US-A-3 260 759**
**BIOCHEMISCHE ZEITSCHRIFT, Bd. 259 1933, Berlin**

(73) Patentinhaber: **BAYER AG, Zentralbereich Patente, Marken und Lizenzen, D-5090 Leverkusen 1, Bayerwerk (DE)**

(72) Erfinder: **Stemmler, Ingo, Dr., Buschweg 21, D-5068 Odenthal (DE)**
Erfinder: **Wagner, Kuno, Dr., Am Kiesberg 8, D-5090 Leverkusen 1 (DE)**
Erfinder: **Schneider, Gottfried, Dr., Paul-Klee-Strasse 62, D-5090 Leverkusen 1 (DE)**
Erfinder: **Ziemann, Heinz, Dr., Am Wiesenberg 10, D-5653 Leichlingen (DE)**

(56) Entgegenhaltungen:
**L. ORTHNER und E. GERISCH «Über die Primärstufen der Kondensation von Formaldehyd, Seiten 30-52**
**IZV. AKAD. NAUK SSR, SER. KHIM. 1969(4), April 1969 Moskau**
**N.A. VASYUNINA, G.S. BARYSHEVA u. A.A. BALANDIN «Kataliticheskie svoistva ruteniya v reaktsiigidrirovaniya monosakharidov» Seiten 848-854**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

EP 0 007 101 B1

# Verfahren zur Herstellung von Polyalkoholen sowie Verfahren zur Herstellung von Polyurethan-Kunststoffen unter Verwendung dieser Polyalkohole

Die vorliegende Erfindung betrifft ein verbessertes Verfahren zur Herstellung von niedermolekularen Polyalkoholen durch katalytische Hydrierung eines Gemisches verschiedener niedermolekularer Hydroxyaldehyde, Hydroxyketone und gegebenenfalls mehrwertiger Alkohole, wie es bei der Selbstkondensation von Formaldehyd entsteht (im folgenden wird ein derartiges Gemisch als «Formose» bezeichnet). Die Erfindung betrifft auch die Verwendung dieser Polyalkohole für die Herstellung von Polyurethankunststoffen.

Seit den Arbeiten von Butlerow und Loew (Ann. 120, 295 (1861) und J. prakt. Chem. 33, 321 (1886)) im vorigen Jahrhundert ist es bekannt, dass sich bei der Selbstkondensation des Formaldehydhydrats (Formosesynthese) unter dem Einfluss basischer Verbindungen, wie z.B. Calcium- oder Bleihydroxid, Hydroxyaldehyde und Hydroxyketone bilden. In der Folge wurde die Formosesynthese immer wieder bearbeitet. Beispielsweise seien in diesem Zusammenhang Pfeil, Chem. Berichte 84, 229 (1951), Pfeil und Schroth, Chemische Berichte 85, 303 (1952), R.D. Partridge und A.H. Weiss, Carbohydrate Research 24, 29-44 (1972), die Formosen aus Glycerinaldehyd bzw. Dioxyaceton nach Emil Fischer, die deutschen Patentschriften 822385, 830951 und 884791, die US-Patentschriften 2121981, 2224910, 2269935 und 2272378 sowie die englische Patentschrift 513708 genannt. Diese bekannten Verfahen des Standes der Technik sind mit gewissen Nachteilen behaftet (schlechte Raum/Zeit-Ausbeuten, gefärbte Nebenprodukte); in jüngster Zeit wurden jedoch von der Anmelderin neue Verfahren entwickelt, nach denen sich in hoher Ausbeute mit gängigen Katalysatoren praktisch farblose und von störenden Nebenprodukten freie Formosen herstellen lassen.

Eines dieser neuen Verfahren besteht darin, dass man die Kondensation des Formaldehydhydrats in Gegenwart von löslichen oder unlöslichen Blei(II)salzen, bzw. an hochmolekulare Träger gebundenen Blei(II)ionen als Katalysator und eines Gemisches aus Hydroxyaldehyden und Hydroxyketonen als Co-Katalysator ablaufen lässt, wie es bei der Kondensation von Formaldehydhydrat entsteht und welches durch folgende Molverhältnisse charakterisiert ist:

Verbindungen mit 3 C-Atomen/Verbindungen mit 4 C-Atomen: 0,5 bis 2,0

Verbindungen mit 4 C-Atomen/Verbindungen mit 5 C-Atomen: 0,2 bis 2,0

Verbindungen mit 5 C-Atomen/Verbindungen mit 6 C-Atomen: 0,5 bis 5,0

wobei der Anteil der Komponenten mit 3 bis 6 C-Atomen mindestens 75 Gew.-%, vorzugsweise mehr als 85 Gew.-%, bezogen auf gesamten Co-Katalysator, beträgt.

Die Reaktionstemperatur liegt dabei im allgemeinen zwischen 70 und 110°C, bevorzugt zwischen 80 und 100°C, und der pH-Wert der Reaktionslösung wird durch kontrollierte Zugabe einer anorganischen oder organischen Base bis zu einem Umsatz von 10-60%, vorzugsweise 30-50%, auf einen Wert von 6,0-8,0, bevorzugt 6,5-7,0 und anschliessend auf einen Wert von 4,0-6,0, bevorzugt 5,0-6,0 eingestellt. Überraschenderweise lässt sich die Produktverteilung der entsprechenden Polyol-, Hydroxyaldehyd- und Hydroxyketongemische durch diese spezielle pH-Führung und durch anschliessende Kühlung bei verschieden hohen Restformaldehydgehalten (0 bis 10 Gew.-%, vorzugsweise 0,5 bis 6 Gew.-%) in reproduzierbarer Weise variieren.

Nachdem man die Selbstkondensation des Formaldehydhydrates durch Kühlen und/oder durch Desaktivierung des bleihaltigen Katalysators mittels Säuren unterbrochen hat, wird der Katalysator und gegebenenfalls auch das in den Produkten enthaltene Wasser entfernt. Hinsichtlich näherer Einzelheiten sei auf DE-A-2639084 und DE-A-2732077 verwiesen.

Eine weitere Möglichkeit, in hoher Raum-Zeit-Ausbeute hochkonzentrierte farblose Formosen herzustellen, besteht gemäss DE-A-2714084 darin, wässrige Formalinlösungen und/oder Paraformaldehyd-Dispersionen in Gegenwart eines löslichen oder unlöslichen Metallkatalysators und eines durch teilweise Oxidation eines zwei- oder mehrwertigen, mindestens zwei benachbarte Hydroxylgruppen aufweisenden Alkohols mit einem Molekulargewicht zwischen 62 und 242 bzw. eines Gemischs derartiger Alkohole dargestellten Co-Katalysators zu kondensieren, wobei man den pH-Wert der Reaktionslösung durch gesteuerte Zufuhr einer Base bis zu einem Umsatz von 5 bis 40% zwischen 6,0 und 9,0 hält und anschliessend bis zum Abbruch der Kondensationsreaktion auf 4,5 bis 8,0 einstellt, so dass er nun um 1,0 bis 2,0 Einheiten tiefer liegt als in der ersten Reaktionsphase, dann die Reaktion bei einem Restgehalt von 0-10 Gew.-% Formaldehyd durch Desaktivierung des Katalysators unterbricht und den Katalysator entfernt.

Qualitativ hochwertige Formosen können auch durch Kondensation von Formaldehyd in Gegenwart eines Metallkatalysators und mehr als 10 Gew.-%, bezogen auf Formaldehyd, eines oder mehrerer zwei- oder mehrwertiger niedermolekularer Alkohole und/oder höhermolekularer Polyhydroxylverbindungen hergestellt werden (siehe DE-A-2714104).

Besonders wirtschaftlich ist es, gemäss einem weiteren noch unveröffentlichten Verfahren der Anmelderin, Formose direkt, d.h. ohne den Umweg über wässrige Formalinlösungen oder Paraformaldehyd, aus Formaldehyd enthaltenden Synthesegasen herzustellen.

Man leitet zu diesem Zweck die Synthesegase, wie sie bei der grosstechnischen Herstellung von Formaldehyd anfallen, bei Temperaturen zwischen 10 und 150°C kontinuierlich oder diskontinuierlich in eine Absorptionsflüssigkeit, welche aus Wasser, ein- oder mehrwertigen niedermolekularen Alkoholen und/oder höhermolekularen Polyhydroxylverbindungen und/oder zur Endiolbildung befähigten Verbindungen als Co-Katalysator und/oder löslichen oder unlöslichen Metallverbindungen, welche gegebenenfalls an hochmolekulare Träger gebunden sind, als Katalysator besteht und einen pH-Wert von 3 bis 10 aufweist, kondensiert den Formaldehyd direkt in situ in der Absorptionsflüssigkeit (gegebenenfalls auch in einem nachgeschalteten Reaktionsrohr bzw. einer nachgeschalteten Rührkesselkaskade), bricht die Selbstkondensation des Formaldehyds bei einem Restformaldehydgehalt im Reaktionsgemisch von 0 bis 10 Gew.-% durch Kühlen und/oder durch Desaktivierung des Katalysators mittels Säuren ab und entfernt schliesslich den Katalysator.

Gemische aus Hydroxyaldehyden, Hydroxyketonen und gegebenenfalls Polyalkoholen, wie sie nach den vorstehend beschriebenen Verfahren oder nach Verfahren des Standes der Technik erhalten werden, müssen für eine Reihe von Verwendungszwecken durch Reduktion der Carbonylgruppen in Gemische von Polyalkoholen umgewandelt werden (derartige, durch Reduktion von Formosen erhaltene Polyolgemische werden im folgenden «Formite» genannt). So gelingt z.B. die Reduktion von Formose direkt aus wässriger Lösung schon bei Raumtemperatur mit Natriumborhydrid (vgl. R.D. Partridge, A.H. Weiss und D. Todd, Carbohydrate Research 24 (1972), 42); sie kann aber z.B. auch auf elektrochemischem Weg erfolgen.

Verfahren zur katalytischen Hydrierung von Hydroxyaldehyden und Hydroxyketonen, speziell von Zuckern, sind bereits in grosser Zahl entwickelt worden. Dabei wird je nach Verfahrensweise mit sehr unterschiedlichen Mengen und Typen von Katalysatoren gearbeitet. N.A. Vasyunina, G.S. Barysheva und A.A. Balandin (Izv. Akad. Nauk SSR, Ler. Khim. 1969 (4), 848/854) zeigen, dass Ruthenium als Katalysator bei der Hydrierung von Glucose und Xylose im sauren, neutralen und alkalischen pH-Bereich wesentlich bessere Ergebnisse als Platin, Palladium oder Nickel liefert. In Gegenwart von 0,5 Gew.-% Ruthenium werden diese Zucker in 20 Minuten bei 100 bis 120 °C und 80 bar $H_2$ quantitativ hydriert.

In DE-A-2 555 856 wird die katalytische Hydrierung von Kohlenhydraten zu Polyalkoholen mittels Ruthenium auf Aluminosilikatton beschrieben. Nach diesen Verfahren können sowohl Aldosen als auch Ketosen mit vier bis sieben C-Atomen hydriert werden.

Ein Verfahren zur Hydrierung von Formose beschreiben L. Orthner und E. Gerisch in Biochem. Ztg. 259, 30 (1933). In 7–8stündiger Reaktion wird eine 4%ige Formoselösung bei 130 °C unter 120 bar Wasserstoffdruck an Raney-Nickel (170 Gew.-% Katalysator, bezogen auf Formose) hydriert. Ein solches Verfahren ist natürlich in jeder Hinsicht wirtschaftlich unbefriedigend.

Nach US-A-2 775 621 müssen die Formaldehydkondensationsprodukte vor der Hydrierung aufwendigen Reinigungsoperationen, z.B. zur Entfernung von Formaldehyd, unterzogen werden. Danach bewirkt ein Restformaldehydgehalt in der Hydrierlösung von mehr als 0,5% eine starke Hemmung des Hydriervorganges und verursacht ausserdem bei den Hydriertemperaturen von ca. 150 °C die Bildung stark gefärbter Zersetzungsprodukte. 210 Teile einer 10%igen wässrigen Formoselösung mit 1% Formaldehyd sind nach 90minütiger Hydrierdauer bei 150 °C und 140 bar $H_2$ an 20 Teilen feuchtem Raney-Nickel nur zu 49% hydriert. Bei diesem Verfahren muss vor der Hydrierung die Lösung auf einen Formosegehalt von 10 bis 20% verdünnt und ausserdem ein pH von 5 bis 7 eingestellt werden. Bei pH-Werten unter 5 ist die Hydriergeschwindigkeit zu gering, und es werden Teile des Katalysators gelöst. Eine Steigerung des pH-Wertes über 7 verursacht offensichtlich Aldoladditionen der Kohlenhydrate und damit eine unkontrollierbare Veränderung in der Zusammensetzung der zu hydrierenden Formose.

Die in der DE-A-26 39 084 für die Hydrierung von Formose genannten Platinmetalle Platin, Rhodium und Palladium, die aus der Zuckerhydrierung bekannt sind, liefern stark gefärbte Reaktionsprodukte mit einem hohen Restcarbonylgehalt.

Weitere Hydrierverfahren sind in den deutschen Patentschriften 705 274, 725 842, 830 951, 888 069 sowie in den US-Patentschriften 2 224 910, 2 269 935, 2 271 083, 2 272 378, 2 276 192, 2 760 983 und 2 775 621 beschrieben. Gemeinsam ist allen diesen Verfahren die Verwendung von Metallkatalysatoren (bevorzugt Co, Ni, Cu). Alle bekannten Verfahren zur Hydrierung von Formose weisen jedoch einen oder mehrere der folgenden Nachteile auf:

Grosser apparativer Aufwand und schwierige Handhabung wegen hoher Wasserstoffdrucke; hoher Katalysatoraufwand, bezogen auf das zu hydrierende Produkt (10–200 Gew.-%); gefärbte Produkte durch lange Hydrierzeiten 1–10 Stunden); Verunreinigung des Hydrierproduktes durch gelöste Anteile des Katalysators; aufwendige Operationen zur Entfernung von Hydrierinhibitoren aus der Formose vor der Hydrierung; Einstellung eines bestimmten pH-Wertes in der Formoselösung vor der Hydrierung.

Aufgabe der Erfindung war es daher, Katalysatoren zu finden, welche eine schnelle Hydrierung von Formosen ohne vorhergehende langwierige Aufarbeitungs-, Reinigungs- oder Modifizierungsoperationen unter schonenden Bedingungen mit möglichst geringem verfahrenstechnischen Aufwand gestatten. Die weiter oben beschriebenen Nachteile bekannter Hydrierverfahren von Formose sollten vermieden werden.

Die Lösung der gestellten Aufgabe bereitete jedoch aus den im folgenden dargelegten Gründen Schwierigkeiten. Formose ist zwar ein Gemisch von Zuckern unterschiedlicher Struktur und Kettenlänge (z.B. Erythrose, Xylose, Glucose u.a.m.), das durch Formaldehydkondensation gebildete Produktgemisch enthält aber nicht nur geradkettige Zucker, sondern daneben auch in

$\alpha$-Stellung bzw. in ~′- und $\alpha'$-Stellung methylolierte Aldosen und Ketosen. Neben diesen verzweigten Zuckern befinden sich im Reaktionsgemisch ausser dem Katalysator der Formosereaktion und der eingesetzten Base noch Methanol, Zuckeralkohole, Ameisensäure, Säuren der allgemeinen Formel $HOH_2C\text{-}(CHOH)_n\text{-}COOH$ mit n = 0–6 sowie Salze dieser Säuren und – je nach Umsetzungsgrad des Formaldehyds – noch mehr oder weniger hohe Restanteile an HCHO.

Ameisensäure und Formiate zerfallen auch in wässriger Lösung an einigen Metall- und Edelmetallkatalysatoren wie Pd, Pt, Rh, Ir und Ru (vgl. Beilsteins Handbuch der Organischen Chemie, 4. Auflage, Springer Verlag Berlin-Heidelberg-New York; 1920, 1929, 1942, 1960, 1975; Band 2, 8 ff; 2 EI, 7 ff; 2 EII, 3 ff; 2 EIII, 4 ff und 2 EIV, 4 ff und dort zitierte Literatur) entsprechend folgenden Gleichungen:

$$(1)\quad HCOOH_{(aq)} \underset{}{\overset{25\,^\circ C}{\rightleftharpoons}} CO_{2(aq)} + H_{2(g)}$$

$G^\circ$ = −85,1 kcal/Mol   −92,3 kcal/Mol   --

$\triangle G_1^\circ$ = −7,2 kcal/Mol

$$(2)\quad HCOOH_{(aq)} \overset{25\,^\circ C}{\rightleftharpoons} CO_{(g)} + H_2O_{(1)}$$

$G^\circ$ = −85,1 kcal/Mol   −32,81 kcal/Mol   −56,69 kcal/Mol

$\triangle G_2^\circ$ = −4,40 kcal/Mol

(Werte aus: M.A. Paul, Principles of Chemical Thermodynamics. McGraw-Hill, New York 1951)

(Werte aus: M.A. Paul, Principles of Chemical Thermodynamics. McGraw-Hill, New York 1951)

Die gegenüber der Ameisensäure thermodynamisch begünstigten Zerfallsprodukte CO und $CO_2$ wirken nach Paul N. Rylander (Catalytic Hydrogenation over Platinum Metals, Academic Press, New York and London 1967, Part I, S. 20/21) gegenüber allen Platinmetallen jedoch als Katalysatorgifte.

Aus der DE-B-1069135 ist die Hydrierung von Traubenzucker, Fruchtzucker, Rohrzucker, Malzzucker und Milchzucker an Ruthenium auf Kohlenstoff oder Aktivkohle und aus der GB-A-1.497.318 die Hydrierung von Stärke-Hydrolysat an Ruthenium auf Ton bekannt. Wegen der beschriebenen Wirkung der Formose-Inhaltsstoffe Formaldehyd und Ameisensäure als Katalysatorgifte schien eine Verwendung rutheniumhaltiger Katalysatoren für die Hydrierung von Formose ausgeschlossen.

Daher ist es besonders überraschend, dass nun ein Verfahren gefunden wurde, nach welchem Formose im pH-Bereich unter 7,5 (bevorzugt 0 bis 7,0, besonders bevorzugt 2,0 bis 7,0) in schneller Reaktion bei Temperaturen unter 220 °C, bevorzugt zwischen 80 bis 210 °C, besonders bevorzugt 100 bis 200 °C, und bei Wasserstoffdrücken über 10 bar, bevorzugt zwischen 50 und 500 bar, besonders bevorzugt zwischen 100 und 300 bar, unter Verwendung von Ruthenium als Katalysator, zu farblosen Polyolgemischen hydriert werden kann. Formosen, die durch bleikatalysierte Formaldehydkondensation erhalten worden waren, müssen vor der erfindungsgemässen Hydrierung weitgehend von Blei befreit werden (= 250 ppm Pb sind empfehlenswert). Dies ist neu und war aus obengenannten Gründen (Vergiftungsgefahr für den Katalysator; s. auch Verfahren der US-A-2775621) nicht vorauszusehen.

Es ist auch als überraschend anzusehen, dass die Komponentenverteilung der nach dem erfindungsgemässen Verfahren erhaltenen Polyolgemische innerhalb des oben angegebenen Bereichs weder von pH noch von der Hydriertemperatur oder der Hydrierzeit abhängt. Es wäre nämlich zu erwarten gewesen, dass vor allem im stark sauren pH-Bereich infolge der dort besonders begünstigten Aldolkondensation die Zusammensetzung des Formits stark von jener neutral oder schwach sauer hydrierter Produkte abweichen würde. Aus hochfunktionellen Formosen werden erfindungsgemäss stets Polyalkoholgemische mit hohen Funktionalitäten (bis über 6) erhalten. Dies ist für eine Reihe von Anwendungsgebieten von besonderem Vorteil.

Gegenstand der vorliegenden Erfindung ist somit ein Verfahren zur Herstellung eines Gemisches von mehrwertigen Alkoholen durch katalytische Hydrierung von Formose bei pH-Werten unterhalb von 7,5, welches dadurch gekennzeichnet ist, dass als Katalysator Ruthenium, gegebenenfalls im Gemisch mit untergeordneten Mengen an Co-Katalysatoren oder Promotoren, eingesetzt wird, wobei der Katalysator gegebenenfalls auf ein Trägermaterial aufgebracht ist.

Das erfindungsgemässe Hydrierverfahren ist auf beliebige Formosen anwendbar, wie sie bei den Verfahren des eingangs zitierten Standes der Technik erhalten werden. Die Formose kann jedoch auch im Gemisch mit bis zu 80 Gew.-% (bezogen auf Gesamtmenge an zu hydrierenden Verbindungen) an anderen künstlichen oder auch natürlichen Zuckern (wie z.B. Glucose, Maltose, Fructose, Saccharose, Lactose etc.) eingesetzt werden. Es ist dabei von Vorteil, dass Formose ein ausgezeichnetes Lösungsmittel bzw. einen Löslichkeitsvermittler für derartige Zucker darstellt.

Vorzugsweise werden für das erfindungsgemässe Hydrierverfahren vollentsalzte Formosen eingesetzt; es ist jedoch auch möglich, solche

Formosen einzusetzen, aus welchen nur die Kationen, insbesondere die als Katalysator für die Formaldehydkondensation dienenden Schwermetallionen, entfernt werden.

Als erfindungsgemäss mitverwendbare künstliche Invertzucker kommen Hydrolysate von beliebigen Di- und/oder Polysacchariden in Frage, z.B. von Rohrzucker, Mischungen von Rohrzukker und Invertzuckern, Hydrolysate von Trehalose, Maltose oder Isomaltose, Hydrolysate von Mais- und Kartoffelstärke und von Pektinstoffen (Amylose und Amylopektine), Cellobiose und Lactose (Milchzucker), Hydrolysate von Galaktose, Glukosemischungen, Raffinose-Hydrolysate, Cellulose-Hydrolysate, Hydrolysate von Dextrinen, gegebenenfalls in Mischung mit nichthydrolysierten Dextrinen, Hydrolysate der Schardinger-Dextrine (cyclische Dextrine), Hydrolysate des Glykogens, Hydrolysate der Glukose-6-Phosphorsäure, Hydrolysate von Glukose-1-Phosphat (Cori-Ester), Fruktose-6-Phosphat, abgebaute Pektinstoffe (Polygalakturonsäuren), abgebaute Glukosamine, Hydrolysate von Melasserückständen etc.

Die Formose kann im erfindungsgemässen Verfahren aber auch im Gemisch mit Aldehyden, Ketonen, Alkanalen sowie ein- oder mehrwertigen, nieder- oder hochmolekularen Alkoholen (die gleichzeitig auch als Lösungsmittel dienen) eingesetzt werden. Insbesondere kann die Formose noch bis zu 5 Gew.-%, vorzugsweise bis zu 2 Gew.-%, an nicht kondensiertem Formaldehyd enthalten.

Als Aldehyde bzw. Alkanale, die im erfindungsgemässen Verfahren gegebenenfalls mitverwendet werden können, kommen insbesondere Acetaldehyd, Propionaldehyd, Butyraldehyd, Isobutyraldehyd sowie deren Methylolderivate in Betracht.

Als Ketone seien Aceton, Methyläthylketon, Diäthylketon, Cyclopentanon, Cyclohexanon, Mesityloxid, Isophoron, Acetophenon, Benzophenon sowie deren Methylolderivate genannt.

Als Lösungsmittel kommt im erfindungsgemässen Verfahren in erster Linie Wasser in Frage; die Formose kann jedoch auch in beliebigen Mono- oder Polyalkoholen gelöst sein. Geeignete Alkohole sind z.B. Methanol, Äthanol, Propanol, Butanol, Isopropanol, Cyclopentanol, Cyclohexanol, 2-Äthoxyäthanol, 2-Propoxyäthanol, 2-Isopropoxyäthanol, 2-Butoxyäthanol, 2-(2-Methoxyäthoxy)-äthanol, 2-(2-Äthoxyäthoxy)-äthanol, 1,2-Bis-(2-hydroxyäthoxy)-äthan, Äthylenglykol, Diäthylenglykol, Triäthylenglykol, Tetraäthylenglykol, 1,2-Propandiol, Isopropylenglykol, 1,3-Propandiol, 1,2-Butandiol, 1,3-Butandiol, 2-Methoxy-1-butanol, 2,3-Butandiol, 1,5-Pentandiol, 2,2-Dimethyl-1,3-propandiol, 1,6-Hexandiol, 2,5-Hexandiol, 2-Methyl-2,4-pentandiol, 3-Methyl-1,5-pentandiol, 3-Methyl-2,4-pentandiol, 2,3-Dimethyl-2,3-butandiol, 2-Methyl-2-propyl-1,3-propandiol, 2,2-Diäthyl-1,3-propandiol, 2-Äthyl-1,3-hexandiol, 2,5-Dimethyl-2,5-hexandiol, 2,2,4-Trimethyl-1,3-pentandiol, 1,3-Diethoxy-2-propanol, 2-Hydroxymethyl-2-methyl-1,3-propandiol, 1,2,6-Hexantriol, 2-Ethyl-2-hydroxymethyl-1,3-propandiol, 2,2-Bis-hydroxy-methyl-1,3-propandiol, Erythrit, Chinit, Mannit, Sorbit und Methylglykosid, sowie Ethoxylierungs- und Propoxylierungsprodukte dieser Alkohole mit einem Molekulargewicht bis ca. 400 und selbstverständlich auch Gemische dieser Alkohole. Besonders bevorzugt sind Ethylenglykol, Glycerin und 1,4-Butandiol.

Erfindungsgemäss können bei der Formosehydrierung – gegebenenfalls im Gemisch mit den obengenannten Alkoholen – jedoch auch Polyhydroxylverbindungen mit einem Molekulargewicht von 400 bis 10000, vorzugsweise 500 bis 6000, mitverwendet werden, welche zweckmässigerweise ebenfalls bei Raumtemperatur flüssig bzw. in der Formoselösung löslich sind, z.B. mindestens zwei, in der Regel 2 bis 8, vorzugsweise aber 2 bis 4, Hydroxylgruppen aufweisende Polyester, Polyether, Polyacetale, Polycarbonate und Polyesteramide, wie sie für die Herstellung von homogenen und von zellförmigen Polyurethanen an sich bekannt sind.

Als Hydrierkatalysator kommt für das erfindungsgemässe Verfahren der Formosehydrierung Ruthenium in Frage. Ruthenium kann sowohl in elementarer Form als auch in Form von chemischen Verbindungen (z.B. Komplexverbindungen) eingesetzt werden.

Erfindungsgemäss können aber auch Gemische von Ruthenium mit anderen Edelmetallen als Co-Katalysator eingesetzt werden. In diesem Falle ist der Co-Katalysator in untergeordneter Menge, beispielsweise im Gewichtsverhältnis Co-Katalysator zu Ruthenium = 1:5, vorhanden. Geeignete Co-Katalysatoren sind z.B. Pd, Pt, Os, Ag und Au bzw. Verbindungen dieser Metalle.

Erfindungsgemäss geeignete Verbindungen der Katalysatormetalle sind insbesondere solche mit stabilen Oxidationsstufen, vorzugsweise Oxide, Oxidhydrate, Hydroxide und Salze organischer oder anorganischer Säuren, daneben aber auch Koordinationsverbindungen oder Komplexverbindungen. Am Beispiel von Ruthenium sollen nachstehend diese Verbindungsklassen – stellvertretend für alle Katalysatormetalle – beschrieben werden:

$Ru(OH)_2$; $Ru(OH)_3$; $RuO_2$; $RuCl_3$; $M_2[RuX_5]$; $M_3[RuX_6]$; $M_2[RuO_4]$; $RuCl_2(PPh_3)_3$; $RuHCl(PPh_3)_3$ oder Gemische $RuCl_3/PPh_3$; $[Ru(NH_3)_6]X3$; $[RuOH(NH_3)_5]X_2$; $[RuY(NH_3)_5]X_2$; $[RuY_2(NH_3)_4]X$ und $[RuCl_3(NH_3)_3]$.

In den obigen Formeln stehen M für ein einwertiges Metall (vorzugsweise ein Alkalimetall), Ph für einen Phenylrest und X und Y für ein Äquivalent eines organischen oder anorganischen Säurerestes, z.B. für ein Halogenatom, für $CH_3COO$ oder $1/2\ SO_4$.

Im allgemeinen wird der Katalysator im erfindungsgemässen Verfahren zusammen mit einem geeigneten Trägermaterial angewandt. Als Träger für den Katalysator kommen sowohl anorganische Materialien wie Kieselgur, Bimsstein, Kieselsäure, Aluminiumoxide, Alkali- und Erdalkalisilikate, Aluminiumsilikate, Montmorillonit, Bentonit, Aluminiumsilikattone, Kaolinit, Zeolithe, Spinelle, Dolomit, Magnesiumsilikate, Titanoxid, Zirkonoxid, Chrom(III)oxid, Eisenoxid, Zinkoxid, Erdalkalisulfate und -carbonate, Siliciumcarbid, Aluminiumphosphat, Borphosphat, Asbest oder (Aktiv-)Kohle jeglicher Art, als auch organische Materialien, z.B. natürlich vorkommende oder synthetische Verbindungen mit hohem Molgewicht wie Seide, Polyamide, Polystyrole, Zellstoff, Polyurethane oder Kationenaustauscherharze (ganz oder teilweise mit Metall beladen) in Betracht. Die Träger müssen natürlich so gewählt werden, dass bei dem pH-Wert der zu hydrierenden Lösung weder Zersetzung noch Auflösung des Trägermaterials erfolgt.

Die Träger für die erfindungsgemäss zu verwendenden Katalysatoren können beliebige Formen haben, welche eine Hydrierung am entweder als Festbett vorliegenden oder im Reaktionsmedium suspendierten Katalysator erlauben.

Das Trägermaterial kann beispielsweise in Form von Kugeln, Strängen, Fäden, Zylindern, Polygonen oder in Pulverform vorliegen. Weiterhin kann auch die Oberfläche des Reaktionsgefässes als Träger dienen.

Erfindungsgemäss geeignete Katalysatoren dieser Art werden z.B. in der DE-A-2 555 856, DE-A-2 536 416, der DE-B 1 082 245 (GB-A-867 689), den US-A-2 868 847 und 3 055 440 sowie von Paul N. Rylander in «Catalytic Hydrogenation over Platinum Metals», Academic Press, New York and London 1967, Kapitel I.1 (Platinum Metal Catalysts) bzw. den dort zitierten Literaturquellen beschrieben. Diese Veröffentlichungen betreffen nicht nur Kontakte mit einer Metallkomponente, sondern auch Mischungen derselben sowie Mischkontakte mit bis zu vier aktiven Komponenten.

Die katalytisch wirkenden Metalle und/oder deren Verbindungen können im erfindungsgemässen Verfahren selbstverständlich auch ohne Träger in feinverteilter Form als «Mohr», «Schwarz» oder als kolloide Suspension eingesetzt werden. Auch Homogenkatalysatoren wie beispielsweise die Ru-Komplexe der US-A-3 935 284 sind erfindungsgemäss geeignet. Neben Komplexen mit niedermolekularen Komplexliganden können auch Edelmetallkomplexe mit langkettigen oligomeren oder polymeren Liganden eingesetzt werden, wie sie beispielsweise in Chem. Tech. 6 (3), 212/214(1976) von E. Bayer und V. Schurig beschrieben werden. Hierbei liegt die aktive Katalysatorform entweder in molekulardisperser Lösung oder kolloid gelöst vor und kann durch Membranfiltration vom Produkt abgetrennt werden. Für wässrige und alkoholische Medien sind «polymer carrier» vom Polyäthertyp oder z.B. durch Phosphinreste substituierte Polyäther bevorzugt.

Die Katalysatormetalle bzw. ihre katalytisch aktive(n) Oxidationsstufe(n) können in trägerfreier Form oder nach Aufbringen auf Träger durch Reduktion ihrer Verbindungen – bevorzugt ihrer Oxide (z.B. Adams-Katalysatoren) – in an sich bekannter Weise mittels Hydrazin, $B_2H_6$, Borwasserstoffaddukten, Alkaliboranaten, Alkalihydriden, $SO_2$ u.a. oder durch Vorhydrierung entweder des trockenen Oxids oder in Wasser (alkalischer, neutraler oder saurer pH) oder auch in nichtwässrigen Lösungsmitteln (wie Alkohole, Formit usw.) erzeugt werden. Diese Reduktion der Katalysatormetallverbindungen kann vorzugsweise ebenso wie eine eventuelle reduktive Aktivierung der metallischen Katalysatoren der Formosehydrierung direkt vorgeschaltet werden und in derselben Apparatur wie die erfindungsgemässe Hydrierung, bevorzugt in dem Lösungsmittel, in welchem die Formose gelöst ist, erfolgen. Eine solche Vorhydrierung des Katalysators in Formit oder in einer Formitlösung hat u.a. den Vorteil, dass das Hydrierprodukt nicht durch zusätzliches Lösungsmittel verdünnt ist. Diese Vorhydrierung erfolgt im übrigen vorzugsweise unter denselben Bedingungen (Druck, Temperatur) wie das erfindungsgemässe Hydrierungsverfahren.

Falls die Aktivierung des Katalysatormetalles bzw. die Erzeugung desselben aus seinen Verbindungen zugleich mit der Hydrierung der Formose in einem Schritt durchgeführt wird, führt dies oft zu einer unerwünschten Verfärbung der Produkte.

Die erfindungsgemäss zu verwendenden Katalysatoren können, wie schon erwähnt, gegebenenfalls auch durch Zusatz eines oder mehrerer Co-Katalysatoren oder Aktivatoren (Promotoren) aktiviert werden. Der Co-Katalysator bzw. Aktivator kann entweder getrennt oder zusammen mit dem Katalysator zur Formose gegeben werden und während der Hydrierung in gelöster oder fester Form vorliegen. Selbstverständlich ist es auch möglich, die aktivierende Substanz zusammen mit dem Katalysator auf dem Trägermaterial zu fixieren.

Das erfindungsgemäss eingesetzte Ruthenium bzw. Gemische mit den oben beschriebenen Co-Katalysatoren enthalten vorzugsweise als Beschleuniger eines oder mehrere der folgenden Elemente in Mengen bis zu 10 Gew.-%, vorzugsweise 0,1 bis 5 Gew.-% (bezogen auf Gesamtmenge an katalytisch aktiver Substanz): Li, Na, K, Mg, Ca, Ba, Be, La, Ce sowie alle seltenen Erden, Ti, Zr, V, Nb, Ta, Cr, Mo, W, Fe, Co, Ni und Cu. Auch diese Aktivatoren können entweder in elementarer Form oder als schwerlösliche Verbindungen vorliegen, wie beispielsweise als Oxide, Sulfate, Silikate, Spinelle, Phosphate, Carbonate (nur bei pH>6) oder Chromate.

Im allgemeinen werden im erfindungsgemässen Verfahren pro Gramm Formose ca. $10^{-2}$ bis $5.10^{-7}$ g, vorzugsweise $5.10^{-3}$ bis $5.10^{-5}$ g, der katalytisch aktiven Komponente (d.h. ohne Trägermaterial gerechnet) angewandt.

Der pH-Wert der Formose sollte beim erfindungsgemässen Hydrierverfahren unter 7,5 (bevorzugt zwischen 0 und 7, besonders bevorzugt zwischen 2 und 7) liegen, braucht aber auf keinen bestimmten Wert innerhalb dieses Bereichs eingestellt zu werden.

Das erfindungsgemässe Verfahren ist kontinuierlich und diskontinuierlich durchführbar. Eine

vorteilhafte Ausführungsform der diskontinuierlichen Verfahrensvariante läuft folgendermassen ab:

In einem Druckreaktor wird die benötigte Menge an Katalysator (z.B. Ruthenium oder Ruthenium enthaltende Mischkatalysatoren auf Trägermaterial) in Wasser oder Formit vorgelegt. Dann wird der Reaktor mit Wasserstoffgas bis zum Betriebsdruck (10 bis 1000 bar, bevorzugt 50 bis 500 bar, besonders bevorzugt 100 bis 300 bar) gefüllt und anschliessend der Katalysator (vorzugsweise bei einer Temperatur über 100 °C) ca. 60 Minuten lang hydriert und damit aktiviert. Zum abgekühlten Gemisch wird die Formoselösung zugegeben und danach wieder Wasserstoff aufgepresst. In 5 bis 60 Minuten wird unter dem gewünschten $H_2$-Druck (vorzugsweise 100 bis 300 bar) von Raumtemperatur auf eine Hydriertemperatur von 60 bis 220 °C, bevorzugt 80 bis 210 °C, besonders bevorzugt 100 bis 200 °C, erwärmt und bei dieser Temperatur noch bis zu 120 Minuten nachhydriert. Es wird ein farbloses Produkt erhalten, welches 0,01 bis 1 Gew.-%, bevorzugt 0,1 bis 0,5 Gew.-%, Restzucker (berechnet als Glucose) enthält.

Nach einer anderen vorteilhaften Variante des diskontinuierlichen Verfahrens kann die Formose auch im Verlauf von etwa 3 bis 60 Minuten zur heissen Katalysatorsuspension (80 bis 220 °C, bevorzugt 100 bis 200 °C) zugepumpt werden, wobei anschliessend noch ca. 5 bis 60 Minuten nachhydriert wird. Die Gesamthydrierdauer für jeden Ansatz beträgt etwa 5 bis 120 Minuten, vorzugsweise 10 bis 60 Minuten.

Nach einer weiteren bevorzugten Ausführungsform wird Formose an dem erfindungsgemässen Katalysator chargenweise durch Pumphydrierung reduziert. Die erste Charge wird wie in der zuletzt beschriebenen Variante des diskontinuierlichen Verfahrens hydriert. Anschliessend wird bei der Hydriertemperatur aus dem Reaktionsgefäss soviel Reaktionsgemisch durch eine Stahlmantelfritte abgedrückt, dass im Reaktor noch ein Teil des Formits (beispielsweise ca. $^1/_3$ des Reaktionsvolumens) zusammen mit dem Katalysator verbleibt. Danach wird – bezogen auf Katalysatorgewicht einschliesslich Träger – die zwei bis hundertfache, vorzugsweise die drei bis zwanzigfache Menge Formoselösung chargenweise langsam zugepumpt (vorzugsweise mit einer Füllgeschwindigkeit von etwa $^1/_6$ des Reaktorvolumens in 2 Minuten bis 2 Stunden, besonders bevorzugt 3 bis 30 Minuten). Nun wird die halbe bis vierfache Zupumpzeit nachhydriert und danach die Menge Reaktionsgemisch abgedrückt, die der zugepumpten Menge Formoselösung entspricht. Anschliessend wird eine neue Charge in den Reaktor gepumpt und mit dieser sowie mit den folgenden genauso verfahren wie mit der zweiten Charge.

Die Hydrierung von Formose an den eingesetzten Kontakten kann naturgemäss auch kontinuierlich in allen nach dem Stand der Technik üblichen Hydrierreaktoren, z.B. im Reaktionsrohr oder in Rührkesselkaskaden erfolgen.

Der pH-Wert der zu hydrierenden Lösung sollte – wie bereits geschildert – vorteilhafterweise zwischen 2,0 und 7,0 liegen. Er kann mit anorganischen oder organischen Säuren oder Basen auf einen beliebigen Wert im obengenannten pH-Bereich eingestellt werden. Als Säuren kommen beispielsweise Schwefelsäure, Phosphorsäure, Oxalsäure, Sulfonsäuren oder Kationenaustauscherharze ($H^+$-Form), als Basen Natriumhydroxid, Kaliumhydroxid, Calciumhydroxid, Bariumhydroxid, organische Basen und Anionenaustauscherharze ($OH^-$-Form) in Betracht.

Das erfindungsgemässe Vefahren zur Herstellung von hydrierter Formose («Formit») bietet gegenüber dem Stand der Technik folgende Vorteile:

1. Die erfindungsgemäss einzusetzenden Katalysatoren ermöglichen eine äusserst wirtschaftliche Hydrierung (geringer Katalysatorverbrauch; kurze Hydrierzeit).

2. Die Hydrierung von Formose lässt sich mit den beanspruchten Katalysatoren ohne grossen technischen Aufwand durchführen, da wegen der relativ niedrigen Drücke keine Spezialapparaturen verwendet und Sicherungsmassnahmen getroffen werden müssen.

3. Das erfindungsgemässe Verfahren liefert hellfarbige Polyolgemische, die für die verschiedenartigsten Anwendungszwecke ohne weiteren Reinigungsschritt eingesetzt werden können. Dabei ist es unerheblich, ob die Formosen in gereinigtem Zustand oder in technischem Reinheitsgrad hydriert werden. Für das erfindungsgemässe Verfahren ist es lediglich erforderlich, den Bleikatalysator zu entfernen. Es ist dabei als besonders überraschend anzusehen, dass Ameisensäure in der Menge, wie sie durch Cannizzaro-Reaktion während der Formosesynthese entsteht, und Restformaldehyd (bis 2 Gew.-%) das Hydrierverfahren in keiner Weise stören.

4. Ein besonderer Vorzug des Verfahrens besteht darin, dass innerhalb eines weiten pH-Bereiches Formosen gleichen Ursprungs und gleicher Zusammensetzung innerhalb kurzer Reaktionszeiten zu Formiten mit praktisch gleicher molekularer Verteilung hydriert werden können.

5. Aus entsprechenden Formosen können somit Formite mit mittleren Funktionalitäten über 6,0 und einem Anteil an $C_2$- bis $C_4$-Polyolen unter 5% und an Polyolen mit 6 und mehr C-Atomen über 85% erhalten werden. Solche hohen Funktionalitäten der Polyole sind für bestimmte Anwendungsgebiete aber besonders wünschenswert, z.B. auf dem Polyurethan-Hartschaumsektor, wo bei Einsatz hochfunktioneller Polyole die Dimensionsstabilität der resultierenden Schaumstoffe gesteigert wird (vgl. K.C. Frisch und J.E. Kresta, «An Overview of Sugars in Urethanes» in ACS Symposium Series 41, American Chemical Society, Washington D.C. 1977, John L. Hickson editor, S. 246/7).

6. Weiterhin ist es besonders vorteilhaft, dass gewünschtenfalls weitere Verbindungen, wie z.B. Alkanale, Alkohole (z.B. $C_1$- bis $C_{23}$-, insbesondere $C_1$- bis $C_8$-Alkohole, wobei diese Alkohole ein-oder mehrwertig sein können), Ketone, Aldehyde oder höhermolekulare Polyole bei der Hydrierung zugegen sein können, da diese Verbindungen bzw. deren Hydrierungsprodukte die Verträglichkeit der Formite gegenüber den beim Polyisocyanat-Polyadditionsverfahren verwendeten Treibmitteln verbessern. Diese Aldehyde, Ketone und Alkanale können in Mengen bis zu 50 Gew.-% (bezogen auf Gesamtmenge der zu reduzierenden Produkte) zugesetzt werden.

7. Weiterhin lassen sich nach dem erfindungsgemässen Verfahren neben Formose andere natürliche und künstliche Zucker hydrieren.

Bevorzugter Verwendungszweck der erfindungsgemäss hergestellten Gemische von mehrwertigen, niedermolekularen Alkoholen ist ihr Einsatz als Polyolkomponente im Polyisocyanat-Polyadditionsverfahren.

Gegenstand der vorliegenden Erfindung ist somit auch ein Verfahren zur Herstellung von gegebenenfalls zellförmigen Polyurethankunststoffen durch Umsetzung von

a) Polyisocyanaten mit

b) mindestens zwei aktive Wasserstoffatome aufweisenden Verbindungen mit einem Molekulargewicht zwischen 32 und 400, gegebenenfalls

c) mindestens zwei aktive Wasserstoffatome aufweisenden Verbindungen mit einem Molekulargewicht zwischen 400 und 10 000 sowie gegebenenfalls

d) Treibmitteln, Katalysatoren und weiteren an sich bekannten Zusatzstoffen,

welches dadurch gekennzeichnet ist, dass als Komponente b), gegebenenfalls nur anteilsweise, erfindungsgemäss hergestellte Gemische niedermolekularer, mehrwertiger Alkohole eingesetzt werden.

Erfindungsgemäss für die Herstellung von Polyurethankunststoffen geeignete Polyisocyanate, aktive Wasserstoffatome aufweisende Verbindungen, Treibmittel, Katalysatoren, Emulgatoren, Schaumstabilisatoren und weitere Zusatzstoffe sind solche der an sich bekannten Art, wie sie beispielsweise in den Deutschen Offenlegungsschriften 2 639 083, 2 639 084, 2 721 186, 2 738 512, 2 738 513, 2 738 532 und 2 738 533 beschrieben werden.

Weitere Beispiele von gegebenenfalls erfindungsgemäss mitzuverwendenden oberflächenaktiven Zusatzstoffen und Schaumstabilisatoren sowie Zellreglern, Reaktionsverzögerern, Stabilisatoren, flammhemmenden Substanzen, Weichmachern, Farbstoffen und Füllstoffen sowie fungistatisch und bakteriostatisch wirksamen Substanzen sowie Einzelheiten über Verwendungs- und Wirkungsweise dieser Zusatzmittel sind im Kunststoff-Handbuch, Band VII, herausgegeben von Vieweg und Höchtlen, Carl-Hanser-Verlag, München 1966, z.B. auf den Seiten 103 bis 113 beschrieben.

Die Reaktionskomponenten werden erfindungsgemäss nach dem an sich bekannten Einstufenverfahren, dem Prepolymerverfahren oder dem Semiprepolymerverfahren zur Umsetzung gebracht, wobei man sich oft maschineller Einrichtungen bedient, z.B. solcher, die in der amerikanischen Patentschrift 2 764 656 beschrieben werden. Einzelheiten über Verarbeitungseinrichtungen, die auch erfindungsgemäss in Frage kommen, werden im Kunststoff-Handbuch, Band VII, herausgegeben von Vieweg und Höchtlen, Carl-Hanser-Verlag, München 1966, z.B. auf den Seiten 121 bis 205 beschrieben.

Bei der Schaumstoffherstellung wird erfindungsgemäss die Verschäumung oft in Formen durchgeführt. Dabei wird das Reaktionsgemisch in eine Form eingetragen. Als Formmaterial kommt Metall, z.B. Aluminium, oder Kunststoff, z.B. Epoxidharz, in Frage. In der Form schäumt das schäumfähige Reaktionsgemisch auf und bildet den Formkörper. Die Formverschäumung kann dabei so durchgeführt werden, dass das Formteil an seiner Oberfläche Zellstruktur aufweist, es kann aber auch so durchgeführt werden, dass das Formteil eine kompakte Haut und einen zelligen Kern aufweist. Erfindungsgemäss kann man in diesem Zusammenhang so vorgehen, dass man in die Form so viel schäumfähiges Reaktionsgemisch einträgt, dass der gebildete Schaumstoff die Form gerade ausfüllt. Man kann aber auch so arbeiten, dass man mehr schäumfähiges Reaktionsgemisch in die Form einträgt, als zur Ausfüllung des Forminneren mit Schaumstoff notwendig ist. Im letztgenannten Fall wird somit unter «overcharging» gearbeitet; eine derartige Verfahrensweise ist z.B. aus den amerikanischen Patentschriften 3 178 490 und 3 182 104 bekannt.

Bei der Formverschäumung werden vielfach an sich bekannte «äussere Trennmittel», wie Siliconöle, mitverwendet. Man kann aber auch sogenannte «innere Trennmittel», gegebenenfalls im Gemisch mit äusseren Trennmitteln, verwenden, wie sie z.B. aus den deutschen Offenlegungsschriften 2 121 670 und 2 307 589 bekannt geworden sind.

Erfindungsgemäss lassen sich auch kalthärtende Schaumstoffe herstellen (vgl. britische Patentschrift 1 162 517, deutsche Offenlegungsschrift 2 153 086).

Selbstverständlich können aber auch Schaumstoffe durch Blockverschäumung oder nach dem an sich bekannten Doppeltransportbandverfahren hergestellt werden.

Die ausschliessliche Umsetzung der erfindungsgemäss zugänglichen Polyhydroxylverbindungen (ohne Mitverwendung anderer gegenüber Isocyanaten reaktiver Komponenten) mit stark elastifizierenden Polyisocyanaten, wie z.B. Polyisocyanaten mit Biuretstruktur (DE-B-1 543 178), führt zu harten, lichtechten, kratz- und lösungsmittelfesten Beschichtungen und Lacken.

Durch basisch oder sauer katalysierte Propoxylierung oder/und Oxyäthylierung der Polyole lassen sich ferner Polyätheralkohole hoher Funktionalität gewinnen, die in hohen OH-Zahl-Bereichen für die Herstellung von harten bzw. halbharten zellförmigen Polyurethankunststoffen und bei niedrigen OH-Zahlen als Ausgangsmaterialien für hochelastische Polyurethanschaumstoffe Verwendung finden. Bezüglich näherer Einzelheiten

der Polyätherherstellung sei auf DE-A 2639083 verwiesen.

Durch Umsetzung der erfindungsgemäss hergestellten Gemische aus mehrwertigen Alkoholen mit mehrwertigen Carbonsäuren der obengenannten Art, z.B. Phthalsäure, Isophthalsäure, Terephthalsäure, Tetra- und Hexahydrophthalsäure, Adipinsäure oder Maleinsäure nach den üblichen Verfahren der Polyesterkondensation, wie sie beispielsweise in Houben-Weyl, Methoden der organischen Chemie, Band XIV 12, S. 40 beschrieben sind, lassen sich stark verzweigte Polyester synthetisieren, die als Zusätze zu Alkydharzen deren Härte verbessern. Die Hydroxylgruppen enthaltenden Polyester, die aus den erfindungsgemäss hergestellten Hydroxylverbindungen synthetisiert werden, sind selbstverständlich ebenfalls als Ausgangskomponente zur Herstellung von Polyurethankunststoffen brauchbar.

Die erfindungsgemäss hergestellten mehrwertigen Alkohole lassen sich auch sehr leicht mit langkettigen, aliphatischen Monocarbonsäuren, wie Capryl-, Caprin-, Laurin-, Myristin-, Palmitin-, Stearin-, Öl-, Linol-, Arachidon-, oder Behensäure, sowie deren Derivaten, wie z.b. den Methyl- oder Äthylestern oder auch den Anhydriden bzw. gemischten Anhydriden zu hydroxylgruppenhaltigen Estern umsetzen. Diese stellen ebenso wie Oxäthylierungsprodukte der Polyole oder auch Umsetzungsprodukte der erfindungsgemäss zugänglichen Polyhydroxylverbindungen mit langkettigen Monoisocyanaten, wie n-Octyl-, n-Decyl-, n-Dodecyl-, Myristyl-, Cetyl- oder Stearylisocyanat zu Carbamidsäureestern (siehe z.B. K. Lindner, Tenside Bd. III, Wissenschaftliche Verlagsgesellschaft Stuttgart, 1964, S. 2336) nichtionogene, oberflächenaktive Verbindungen dar, die als wertvolle Emulgatoren, Netzmittel oder Weichmacher Verwendung finden können. Die erfindungsgemässen Verbindungen lassen sich auch als Feuchthaltemittel in Kosmetika und Kunststoffen verwenden. Sie können aber z.B. auch als Gefrierschutzmittel dienen oder als Formulierhilfsmittel auf dem Pflanzenschutzsektor.

Die folgenden Beispiele erläutern das erfindungsgemässe Verfahren. Wenn nicht anders vermerkt, sind Mengenangaben als Gewichtsteile bzw. Gewichtsprozente zu verstehen.

Beispiel 1

Diskontinuierliche Hydrierung von Formose an Ruthenium

In einem 3 l Rührautoklaven werden 45 g Katalysator(5% Ruthenium auf Kohle), suspendiert in 500 g Wasser, bei 125 °C und 200 bar $H_2$ innerhalb von 60 Minuten durch Hydrieren aktiviert. Nach Abkühlen der Suspension werden 1500 g einer 30%igen wässrigen Lösung einer vollentsalzten Formose A, hergestellt nach DOS 2639084 (19,8 Gew.-% reduzierende Zucker, berechnet als Glukose; pH = 6), zugegeben. Bei einem Anfangsdruck von 150 bar $H_2$ wird das Gemisch über 30 Minuten auf die gewünschte Temperatur Z = 125 °C erwärmt. Bei Z °C und 200 bar $H_2$ wird noch 90 bis 120 Minuten weiterhydriert. Während der Aufheizphase werden alle 10 Minuten bei t °C, danach noch alle 15 Minuten (Z °C) Proben entnommen. Durch Heissreduktion von Fehling'scher Lösung wird der Restzuckergehalt dieser Proben – berechnet als Glukose – bestimmt. Als Hydrierprodukt erhält man eine farblose Lösung von Polyalkoholen, welche nach Abfiltrieren vom Katalysator zu einer sirupartigen Masse im Vakuum eingeengt wird. Weitere Daten: s. Tabelle 1.

Beispiele 2 bis 10

Es wird wie in Beispiel 1 verfahren, jedoch ausser Formose A werden auch die nach DE-A-2721186 hergestellten, von Kationen befreiten Formosen B (50%ige wässrige Lösung; 34,3% reduzierende Zucker, berechnet als Glukose; pH = 5), C (50%ige wässrige Lösung; 34,3% Zucker; pH = 4) und D (45%ige wässrige Lösung, 30,4% Zukker; pH = 2,8) hydriert.

Als Trägermaterialien für den Rutheniumkatalysator werden getestet: verschiedene Kohlearten (trocken und wasserhaltig), α- und γ-$Al_2O_3$ sowie Y-Zeolith ($Na^+$- und $H^+$-Form). Die Hydriertemperaturen und die pH-Werte der Formoselösungen werden variiert. Weiteres s. Tabelle 1.

Die verwendeten Formosen A bis D wiesen die folgende, durch Gaschromatographie von hydrierten und silylierten Proben bestimmte Komponentenverteilung auf:

| Formose | $C_2$ | $C_3$ | $C_4$ | $C_5$ | $C_6$ | $C_7$ | $C_8$ |
|---|---|---|---|---|---|---|---|
| A | 0,05 | 2,4 | 6,6 | 31,1 | 32,8 | 17,6 | 9,3 |
| B | 0,6 | 1,0 | 2,7 | 14,0 | 39,8 | 32,8 | 9,1 |
| C | 0,03 | 0,4 | 1,3 | 11,8 | 41,8 | 35,5 | 9,3 |
| D | 0,15 | 0,6 | 1,3 | 11,5 | 39,3 | 37,6 | 9,5 |

Tabelle 1

| Beispiel | Trägermaterial | g aktives Metall/g Formose | Anfangs-pH | Formose | Restzucker [%Glukose]/Temperatur [°C] Zeit [Min]: 0 | 10 | 20 | 30 | 45 | 60 | 75 | 90 | 105 |
|---|---|---|---|---|---|---|---|---|---|---|---|---|---|
| 1 | Kohle | $5\times10^{-3}$ | 6,0 | A | 15/42 | 3,3/68 | 1,5/100 | 0,4/125 | 0,08 | 0,04 | 0,03 | 0,03 | 0,03 |
| 2 | $\gamma$-$Al_2O_3$ | $5\times10^{-3}$ | 6,0 | A | 15/30 | 4,9/90 | 2,0/110 | 0,4/125 | 0,05 | | | | |
| 3 | $\alpha$-$Al_2O_3$ | $5\times10^{-3}$ | 6,0 | A | 15/40 | 4,9/85 | 0,7/105 | 0,3/125 | 0,09 | 0,06 | 0,04 | 0,04 | 0,03 |
| 4 | Y-Zeolith | $5\times10^{-3}$ | 6,0 | A | 15/40 | 11,1/70 | 2,1/100 | 0,3/125 | 0,07 | 0,05 | 0,04 | 0,03 | 0,03 |
| 5 | Kohle | $2,9\times10^{-3}$ | 5,0 | B | 25,8/40 | 19,4/73 | 13,2/102 | 4,9/125 | 0,6 | 0,3 | 0,2 | 0,1 | 0,1 |
| 6 | Kohle | $2,9\times10^{-3}$ | 5,0 | B | 25,8/40 | 18,0/76 | 13,0/108 | 2,2/140 | 0,4 | 0,1 | 0,1 | 0,07 | 0,08 |
| 7 | Kohle | $2,9\times10^{-3}$ | 5,0 | B | 25,8/40 | 19,2/90 | 4,7/130 | 1,0/160 | 0,1 | 0,1 | 0,1 | 0,1 | 0,09 |
| 8 | Y-Zeolith ($H^+$-Form) | $2,9\times10^{-3}$ | 4,0 | C | 25,8/22 | - | - | 0,3/125 | 0,2 | 0,1 | 0,1 | 0,1 | 0,06 |
| 9 | Kohle (66,5% $H_2O$) | $2,9\times10^{-3}$ | 4,0 | C | 25,8/45 | 9,5/65 | 5,1/90 | 0,6/125 | 0,0 | | | | |
| 10 | Kohle (66,5% $H_2O$) | $3,3\times10^{-3}$ | 2,8 | D | 22,8/40 | 20,7/70 | 9,1/100 | 1,5/125 | 0,3 | 0,1 | 0,08 | 0,08 | 0,07 |

In allen Beispielen 1 bis 10 war die Komponentenverteilung des erfindungsgemäss erhaltenen Hydrierungsproduktes (bestimmt durch Gaschromatographie an silylierten Proben) innerhalb der Messfehler identisch mit der Komponentenverteilung der jeweiligen Ausgangsformose.

Beispiel 11 bis 16
Chargenweise
Pumphydrierung von Formose an Ru

1. Zyklus
In 500 g Wasser werden 134 g Katalysator (5% Ruthenium auf Kohle mit 66,5% Wassergehalt) bei 125°C und 200 bar $H_2$ im Rührautoklaven 1 Stunde lang vorhydriert. Im Verlauf von a Minuten werden 1500 g einer 60%igen wässrigen Lösung von Formose D bei t°C und 20 bar $H_2$ zugepumpt. Anschliessend wird noch b Minuten bei t°C und 200 bar $H_2$ weiterhydriert; danach wird die erste Charge von ca. 500 g aus dem Reaktionsgefäss durch eine Tauchfritte herausgedrückt.

2. Zyklus und folgende
Über c Minuten werden 500 g einer 45%igen wässrigen Lösung von Formose D bei t°C und 200 bar $H_2$ zugepumpt. Danach wird noch d Minuten bei t°C und 200°C nachhydriert und die hydrierte Charge aus dem Autoklaven herausgepresst. Anschliessend wird erneut zugepumpt.

Hydrierdaten und analytische Daten siehe Tabelle 2.

Tabelle 2

| Beispiel | pH | t [°C] | Pumpzeit a | [min] c | Nachhydrierzeit [min] b | d | Charge Nr.: | 1 | 2 | 3 | 4 | 5 | 6 | 7 | 8 | 9 | 10 | 11 | 12 |
|---|---|---|---|---|---|---|---|---|---|---|---|---|---|---|---|---|---|---|---|
| 11 | 2,8 | 125 | - | 15 | 45 | 15 | | 0,5 | 0,2 | 0,2 | 0,1 | 0,1 | 0,2 | 0,2 | 0,2 | 0,2 | 0,3 | 0,3 | |
| 12 | 2,8 | 140 | 45 | 10 | 30 | 10 | | 0,1 | 0,1 | 0,1 | 0,2 | 0,2 | 0,2 | 0,2 | 0,2 | 0,2 | 0,2 | 0,3 | 0,2 |
| 13 | 2,8 | 160 | 30 | 5 | 30 | 5 | | 0,1 | 0,2 | 0,2 | 0,2 | 0,2 | 0,2 | 0,2 | 0,2 | 0,2 | 0,2 | 0,2 | |
| 14 | 2,8 | 180 | 20 | 4 | 20 | 1 | | 0,2 | 0,3 | 0,4 | 0,3 | 0,4 | 0,5 | 0,6 | 0,7 | 0,7 | | | |
| 15 | 2,8 | 200 | 20 | 3 | 20 | 3 | | 0,2 | 0,2 | 0,2 | 0,3 | 0,3 | 0,3 | 0,3 | 0,2 | 0,3 | 0,4 | 0,2 | |
| | | | | | | | Charge Nr.: | 1 | 2 | 3 | 4 | 5 | 10 | 20 | 30 | 40 | 50 | 70 | 100 |
| 16 | 4,4 | 160 | 8 | 8 | 12 | 12 | | 0,2 | 0,2 | 0,3 | 0,2 | 0,3 | 0,2 | 0,2 | 0,2 | 0,2 | 0,2 | 0,2 | 0,3 |

Beispiel 17
In einem 3 l-Rührautoklaven werden 315 g Katalysator (Ruthenium auf Kohle mit 71,4% Wassergehalt), suspendiert in 500 g Wasser, 1 Stunde bei 160°C und 200 bar $H_2$ vorhydriert. Zum abgekühlten Gemisch werden 1500 g einer 50%igen wässrigen Lösung von Formose D gegeben, und im Verlauf von 30 Minuten wird bei einem Anfangsdruck von 150 bar $H_2$ auf 160°C aufgeheizt. Danach wird noch 120 Minuten bei 160°C und 200

bar $H_2$ hydriert. Nach 60 Minuten Gesamt-Reaktionszeit enthält das Reaktionsprodukt noch 0,1% Restzucker (berechnet als Glukose).

Beispiel 18

Hydrierung von Formose an Ruthenium in Gegenwart von Ameisensäure

Man verfährt wie in Beispiel 17, setzt jedoch zu den 1500 g Formose D noch 15 g reine Ameisensäure zu. Während der Hydrierung werden Proben von jeweils 1 g entnommen und der Säuregehalt mit 0,1 n Natronlauge titrimetrisch bestimmt.

| Hydrierzeit [min] | 10 | 20 | 30 | 45 | 60 | 75 |
|---|---|---|---|---|---|---|
| 0,1 n Natronlauge [ml] | 1,6 | 1,5 | 1,4 | 0,4 | 0,2 | 0,2 |

Nach 60 Minuten (einschliesslich Aufheizphase) enthält das Hydrierprodukt noch 0,1% Restzucker (ber. als Glukose).

Beispiel 19

Hydrierung von formaldehydhaltiger Formose an Ruthenium

Man führt die Hydrierung wie in Beispiel 17 durch, verwendet jedoch 1500 g Formose D, welche 37 g Formaldehyd enthält. Nach 60 Minuten Gesamthydrierzeit enthält das Reaktionsprodukt nur noch 0,3% Zucker (berechnet als Glukose); Formaldehyd kann nicht mehr nachgewiesen werden.

Beispiel 20

Herstellung eines Polyurethanschaumstoffes

21,6 Teile eines Gemisches aus 3 Teilen des Formits aus Beispiel 16 und 1 Teil des Adduktes aus 1 Mol Wasser und 1 Mol ε-Caprolactam,
20,4 Teile eines Propoxylierungsproduktes von Äthylendiamin (OH-Zahl = 450),
7,0 Teile Tris-β-chloräthylphosphat,
18,7 Teile Monofluortrichlormethan,
0,5 Teile Dimethylbenzylamin,
0,75 Teile eines handelsüblichen Silikonstabilisators (L-5420 der UCC) und
81 Teile eines technischen Phosgenierungsproduktes von Anilin/Formaldehyd-Kondensaten (NCO-Gehalt: 29%)
werden intensiv vermischt und das Gemisch in einer offenen Form aufschäumen gelassen. Nach dem Aushärten bei 80°C erhält man einen harten, feinzelligen Schaumstoff mit grosser Reissfestigkeit und Dimensionsstabilität.

Beispiel 21 (Vergleichsbeispiel)

Versuch zur Hydrierung von Formose an Rhodium/Kohle. 45 g Rhodium-Kohle-Katalysator (5% Rh) werden in 500 g Wasser bei 160°C und 200 bar $H_2$ 1 Stunde vorhydriert. Zur abgekühlten Suspension werden 1500 g einer nach DE-A-27 21 186 hergestellten, über Ionenaustauscher entsalzten 45%igen wässrigen Formoselösung gegeben. Während 30 Minuten wird bei einem Anfangsdruck von 150 bar $H_2$ von Raumtemperatur auf 160°C unter Rühren erwärmt und anschliessend noch weitere 150 Minuten bei 160°C und 200 bar $H_2$ hydriert. Der Zuckergehalt (ber. als Glukose) des Reaktionsgemisches ist von anfangs 22,9% nur auf 8,0% gesunken. Als Reaktionsprodukt wird eine braune Lösung erhalten.

Beispiel 22 (Vergleichsbeispiel)

Hydrierung von Formose an Ruthenium/Kohle im Gemisch mit Palladium/Kohle (Gewichtsverhältnis Ru:Pd = 1:5). 9 g Ruthenium/Kohle-Katalysator (5% Ru) und 225 g Palladium-Kohle-Katalysator (1% Pd) werden in 500 g Wasser bei 125°C und 200 bar $H_2$ 1 Stunde vorhydriert. Nach Zusatz von 1500 g der nach DE-A-27 21 186 hergestellten Formoselösung wird unter Rühren bei einem Anfangsdruck von 150 bar $H_2$ innerhalb 30 Minuten auf 125°C erwärmt. Danach wird noch 120 Minuten bei 125°C und 200 bar $H_2$ weiter hydriert. Das Reaktionsprodukt enthält noch immer 13,2% Restzucker (ber. als Glukose).

Beispiel 23

Hydrierung von Formose an einem Katalysatorgemisch Ruthenium/Kohle und Palladium/Kohle (Gewichtsverhältnis Ru:Pd = 5:1). 45 g Ruthenium/Kohle-Katalysator (5% Ru) und 45 g Palladium/Kohle-Katalysator (1% Pd) werden wie in Beispiel 2 durch Vorhydrieren aktiviert. Unter gleichen Reaktionsbedingungen wie in Beispiel 2 werden anschliessend 1500 g der genannten Formoselösung hydriert. Nach 30 Minuten Aufheizphase und 60 Minuten Hydrierdauer enthält das Reaktionsprodukt nur noch 0,2%, nach weiteren 15 Minuten nur noch 0,1% Restzucker (ber. als Glukose).

**Patentansprüche**

1. Verfahren zur Herstellung eines Gemisches von mehrwertigen Alkoholen durch katalytische Hydrierung von Formose, dadurch gekennzeichnet, dass als Katalysator Ruthenium, gegebenenfalls im Gemisch mit untergeordneten Mengen an Co-Katalysatoren oder Promotoren eingesetzt wird.

2. Verfahren nach Anspruch 1, dadurch gekennzeichnet, dass der Katalysator vor der Hydrierung der Formose in Gegenwart von reduzierter Formose im Reduktionsreaktor mit Wasserstoff unter Reduktionsbedingungen aktiviert wird.

3. Verfahren nach Anspruch 1 bis 2, dadurch gekennzeichnet, dass $10^{-2}$ bis $5.10^{-7}$ g Katalysator pro g Formose eingesetzt werden.

4. Verfahren nach Anspruch 1 bis 3, dadurch gekennzeichnet, dass bei einem pH-Wert zwischen 2,0 und 7,0 hydriert wird.

5. Verfahren nach Anspruch 1 bis 4, dadurch gekennzeichnet, dass die zu hydrierende Formose bis zu 5 Gew.-% an Formaldehyd und/oder bis zu 80 Gew.-%, bezogen auf Gesamtmenge an zu hydrierenden Produkten, an weiteren natürlichen oder synthetischen Zuckern enthält.

6. Verfahren zur Herstellung von gegebenenfalls zellförmigen Polyurethankunststoffen durch

Umsetzung von
a) Polyisocyanaten mit
b) mindestens 2 aktive Wasserstoffatome aufweisenden Verbindungen mit einem Molekulargewicht zwischen 32 und 400, gegebenenfalls
c) mindestens 2 aktive Wasserstoffatome aufweisenden Verbindungen mit einem Molekulargewicht zwischen 400 und 10 000 sowie gegebenenfalls
d) Treibmitteln, Katalysatoren und weiteren an sich bekannten Zusatzstoffen,
dadurch gekennzeichnet, dass als Komponente b), gegebenenfalls nur anteilsweise, gemäss Anspruch 1 bis 5 hergestellte Gemische niedermolekularer, mehrwertiger Alkohole, eingesetzt werden.

**Patent Claims**

1. Process for the preparation of a mixture of polyhydric alcohols by the catalytic hydrogenation of formose, characterised in that ruthenium, optionally in admixture with lower amounts of cocatalysts or promoters, is used as the catalyst.

2. Process according to claim 1, characterised in that prior to the hydrogenation of formose the catalyst is activated with hydrogen under reduction conditions in the reduction reactor in the presence of reduced formose.

3. Process according to claim 1 to 2, characterised in that from $10^{-2}$ to $5 \times 10^{-7}$ g of catalyst are used per g of formose.

4. Process according to claim 1 to 3, characterised in that hydrogenation is carried out at a pH of from 2.0 to 7.0.

5. Process according to claim 1 to 4, characterised in that the formose to be hydrogenated contains up to 5% by weight of formaldehyde and/or up to 80% by weight, based on the total quantity of products to be hydrogenated, of other natural or synthetic sugars.

6. Process for the production of optionally cellular polyurethanes by the reaction of
a) polyisocyanates with
b) compounds with a molecular weight of from 32 to 400 having at least two active hydrogen atoms, optionally
c) compounds with a molecular weight of from 400 to 10,000 having at least 2 active hydrogen atoms, and optionally
d) blowing agents, catalysts and other known additives, characterised in that component b) consists partly or completely of mixtures of low molecular, polyhydric alcohols prepared according to claim 1 to 5.

**Revendications**

1. Procédé de préparation d'un mélange d'alcools polyvalents par hydrogénation catalytique de formose, caractérisé en ce que, comme catalyseur, on utilise du ruthénium, éventuellement en mélange avec de faibles quantités de cocatalyseurs ou de promoteurs.

2. Procédé suivant la revendication 1, caractérisé en ce que, avant l'hydrogénation du formose, on active le catalyseur en présence de formose réduit dans un réacteur de réduction avec de l'hydrogène et dans des conditions réductrices.

3. Procédé suivant l'une quelconque des revendications 1 et 2, caractérisé en ce qu'on utilise $10^{-2}$ à $5.10^{-7}$ g de catalyseur par g de formose.

4. Procédé suivant l'une quelconque des revendications 1 à 3, caractérisé en ce qu'on effectue l'hydrogénation à un pH se situant entre 2 et 7.

5. Procédé suivant l'une quelconque des revendications 1 à 4, caractérisé en ce que le formose à hydrogéner contient jusqu'à 5% en poids de formaldéhyde et/ou jusqu'à 80% en poids (calculés sur la quantité totale des produits à hydrogéner) d'autres sucres naturels ou synthétiques.

6. Procédé de préparation de matières synthétiques de polyuréthanes éventuellement cellulaires en faisant réagir:
a) des polyisocyanates, avec
b) des composés comportant au moins 2 atomes d'hydrogène actif et ayant un poids moléculaire compris entre 32 et 400, éventuellement
c) des composés comportant au moins 2 atomes d'hydrogène actif et ayant un poids moléculaire compris entre 400 et 10.000, ainsi qu'éventuellement
d) des agents moussants, des catalyseurs et d'autres additifs connus en soi,
caractérisé en ce que, comme composant (b), on utilise, éventuellement par portions uniquement, des mélanges d'alcools polyvalents de faible poids moléculaire, ces mélanges étant préparés par le procédé suviant les revendications 1 à 5.